# EUROPEAN PATENT APPLICATION

(11) **EP 2 223 655 A1**
(43) Date of publication of application: **01.09.2010**
(21) Application number: 09177802.7
(22) Date of filing: 02.12.2009
(51) Int. Cl.: A61B 8/08, G01S 7/521, G10K 11/00

(54) **Ultrasonic diagnosis device**

(30) Priority: 27.02.2009 KR 20090016949
(71) Applicant: MEDISON CO., LTD., Gangwon-do 250-875 (KR)
(72) Inventor: Joo, Jongho, 137-848, Seoul (KR)
(74) Representative: Schmid, Wolfgang

(57) **Abstract**

Provided is an ultrasonic diagnostic device including a probe. When an ultrasonic diagnosis is performed, the ultrasonic diagnostic device may limit an elastic movement of an inspection portion of a subject occurring due to a contact pressure of the probe by disposing a movement limiting instrument on the inspection portion of the subj ect.

## Description

### BACKGROUND

### 1. Field of the Invention

The present invention relates to an ultrasonic diagnostic device including a probe, and more particularly, to an ultrasonic diagnostic device that may perform an ultrasonic diagnosis for a subject elastically moving due to a contact pressure of the probe.

### 2. Description of the Related Art

Generally, an ultrasonic diagnostic device may transmit sound waves, for example, 2 MHz through 20 MHz of an inaudible frequency band for a human being, that is, may transmit ultrasonic signals to a subject, and receive the ultrasonic signals reflected from the subject to thereby visualize internal tissue of the subject. Since the ultrasonic waves have different reflectional properties at a boundary surface of two different materials, it is possible to obtain an image of the internal tissue.

The ultrasonic diagnostic device may include a probe to transmit and receive the ultrasonic signals to and from the subject, and to receive response signals reflected from the subject. The ultrasonic diagnostic device may reconstruct the received response signals and create an image of an internal tissue with respect to an inspection portion of the subject. The created image may be displayed on a monitor of the ultrasonic diagnostic device. A user may review the image of the internal tissue of the subject displayed on the monitor. Due to the above aspect, the ultrasonic diagnostic device is being widely used in the medical field to accurately diagnose afflictions of a patient.

The inspection portion of the subject may be formed of an elastically moving material. In this case, when the probe is attached onto the subject, the inspection portion of the subject may elastically move due to a contact pressure of the probe. Specifically, when the probe is vertically attached onto the subject, the inspection portion of the subject may move in a direction orthogonal to the attached direction of the probe.

When an abnormal elastic transformation occurs in tissue of the subject, internal tissue information of the subject detected by the ultrasonic diagnostic device may be distorted. Accordingly, inaccurate image information may be displayed on the monitor of the ultrasonic diagnostic device. In addition, a reliability and accuracy of the ultrasonic diagnostic device may be deteriorated.

In particular, when the inspection portion of the subject is very fluidic such as a breast, the abnormal elastic movement occurring in the inspection portion of the subject may become worse. Accordingly, the reliability and accuracy of the ultrasonic diagnostic device may be further deteriorated. Also, an erroneous diagnosis probability may increase.

### SUMMARY

An aspect of the present invention provides an ultrasonic diagnostic device that may prevent an abnormal elastic movement of an inspection portion of a subject occurring due to a contact pressure of a probe attached onto the subject, when an ultrasonic diagnosis is performed.

Another aspect of the present invention also provides an ultrasonic diagnostic device that may enhance an inspection scheme of a probe and thereby compensate for a distortion of an image occurring due to an abnormal elastic movement of an inspection portion of a subject.

Another aspect of the present invention also provides an ultrasonic diagnostic device that may prevent an abnormal tissue transformation of an inspection portion of a subject occurring due to a contact pressure of a probe, and thereby may enhance a reliability and accuracy of an ultrasonic diagnostic device and may also decrease an erroneous diagnosis probability when an ultrasonic diagnosis is performed.

According to an aspect of the present invention, there is provided an ultrasonic diagnostic device including: a body inspecting a subject using an ultrasonic signal; a probe being connected with the body to contact with an inspection portion of the subject, and to transmit and receive the ultrasonic signal to and from the subject; and a movement limiting instrument being disposed on the inspection portion of the subject to limit an abnormal elastic movement of the inspection portion of the subject occurring due to a contact pressure of the probe, when an ultrasonic inspection is performed for the subj ect.

Specifically, the abnormal elastic movement of the inspection portion of the subject may be limited by the movement limiting instrument. Therefore, even when the probe is attached onto the subject at a high pressure, the inspection portion of the subject may be maintained in a predetermined shape. Through a simple configuration change of adding the movement limiting instrument, it is possible to stably enhance a reliability and accuracy of the ultrasonic diagnostic device.

The movement limiting instrument may have a section in a circular shape, an oval shape, a polygonal shape, or a closed-curved shape. A subject opening may be formed in a lower portion of the movement limiting instrument, and the inspection portion of the subject is inserted into the subject opening. A probe opening may be formed in an upper portion of the movement limiting instrument to inspect the inspection portion of the subject inserted into the subject opening using the probe.

Accordingly, the movement limiting instrument may be provided on the inspection portion of the subject to insert the inspection portion of the subject into the subject opening. Also, the probe may be provided within the probe opening to inspect the inspection portion of the subject disposed within the movement limiting instrument.

The movement limiting instrument may have a section in a circular shape, an oval shape, a polygonal shape, or a closed-curved shape. For example, the section of the movement limiting instrument may be formed in a circular shape, an oval shape, a triangular shape, a rectangular shape, or a pentagonal shape. Also, the section of movement limiting instrument may be provided in various shapes optimized for the inspection portion of the subject.

For example, when the section of the movement limiting instrument is formed in the rectangular shape, a shape of the probe opening may also be provided in the rectangular shape. Also, movement coordinates of the probe may be easily set. Therefore, it is possible to more easily design an image creation algorithm of the ultrasonic diagnostic device.

The movement limiting instrument may be formed of a solid material to stably limit an elastic movement of the inspection portion of the subject. For example, the movement limiting instrument may be formed of a metal, a plastic, a glass, or a wood.

The body may include: a transceiver controlling the ultrasonic signal transmitted from the probe, and receiving a response signal received by the probe; a detector detecting image information associated with the inspection portion of the subject from the response signal transferred from the transceiver; and a creator to create an image of an internal tissue of the subject from the detected image information.

The transceiver may control a magnitude, a direction, a time, and the like of the ultrasonic signal transmitted from the probe. The transceiver may receive the response signal and transfer the response signal to the detector. Here, the response signal denotes the ultrasonic signal reflected from the internal tissue of the subject.

The detector may detect image information associated with the inspection portion of the subject from the response signal. The creator may realize an image based on the image information of the subject, and may transfer the created image to a display unit.

A plurality of scan lines that is extended along a contact path of the probe may be formed on the inspection portion of the subject. The plurality of scan lines may be disposed in parallel at regular intervals. However, the present invention is not limited thereto. Specifically, the plurality of scan lines may be disposed at different intervals as necessary. The scan lines may correspond to portions where the ultrasonic signals are transmitted to and are received from the subject. Accordingly, the ultrasonic diagnosis may be performed for the subject based on the scan lines.

Also, the detector may set the plurality of scan lines to have different sizes of inspection areas in order to compensate for the abnormal elastic movement of the inspection portion of the subject. For example, to compensate for a distortion in image information occurring due to the elastic movement of the inspection portion of the subject, the detector may adjust at least one inspection area among the inspection areas of the scan lines. Inspection areas of each of the scan lines may be formed on both sides thereof, and may also formed on one side of the scan lines.

Hereinafter, the above technical description will be further described in detail. When the ultrasonic diagnosis is performed, ultrasonic signals may be transmitted from the probe along the scan lines. Response signals received from the probe may be transferred to the transceiver. The transferred response signals may be transferred from the transceiver to the detector. The detector may detect image information associated with the internal tissue of the subject from the response signals.

When the detector uses all the image information associated with the inspection portion of the subject, an amount of data of the response signals may significantly increase and thus a data processing time may also significantly increase. Accordingly, the image associated with the inspection portion of the subject may not be displayed in real time and be delayed for a long period of time. Also, since the detector requires a high performance calculation device, costs of the ultrasonic diagnostic device may increase. Accordingly, the ultrasonic diagnostic device may selectively adjust the inspection areas of the scan lines to decrease the number of data that need to be processed by the detector. In addition, the ultrasonic diagnostic device may estimate and approximate image information associated with other areas excluding the inspection areas of the scan lines, based on the image information of the inspection areas using various types of schemes.

When the probe is tightly attached onto the inspection portion of the subject, the inspection portion of the subject may be pressed by the probe and the abnormal elastic movement may occur from a center of the probe towards a side direction. The abnormal elastic movement of the inspection portion of the subj ect may be very small in the center of the probe, however, may become greater as the inspection portion of the subject is further from the center of the probe. When the movement limiting instrument is disposed on the inspection portion of the subject, the movement limiting instrument may significantly decrease the abnormal elastic movement of the inspection portion of the subject. Even in this case, the elastic movement of the subject may slightly occur within the movement limiting instrument.

Accordingly, the detector may set the scan lines to have different sizes of inspection areas. Through this, when calculating image information of the subject, it is possible to decrease an error and distortion occurring due to the abnormal elastic movement of the inspection portion of the subject. For example, a relatively greater abnormal elastic movement of the inspection portion of the subject may occur as the inspection portion of the subject is further from the center of the probe. Therefore, the inspection areas of the scan lines may be extended in proportion to distances between the center of the probe and the scan lines. Specifically, as the inspection areas of the scan lines are extended with being further from the center of the probe, an inspection area of a portion where the great amount abnormal elastic movement of the inspection portion of the subject occurs may increase. Accordingly, it is possible to effectively compensate for a displacement of the subject according to an elastic movement amount of the inspection portion of the subject. Also, since the inspection areas of the scan lines are limited, it is possible to prevent a significant increase in the number of data processed by the detector.

The scan lines may be symmetrically disposed on the left and the right of the probe based on the center of the probe. Accordingly, sizes of the inspection areas of the scan lines may be symmetrically formed on the left and the right of the probe based on the center of the probe.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
- FIG. 1: is a diagram illustrating a configuration of an ultrasonic diagnostic device according to an embodiment of the present invention;
- FIG. 2: is a view for describing an operational state when the ultrasonic diagnostic device of FIG. 1 is used without a movement limiting instrument;
- FIG. 3: is a view for describing a disposed state of the movement limiting instrument when the ultrasonic diagnostic device of FIG. 1 is used with the movement limiting instrument;
- FIG. 4: is a view for describing an operational state when the ultrasonic diagnostic device of FIG. 1 is used with the movement limiting instrument;
- FIG. 5: is a perspective view for describing another example of the movement limiting instrument of FIG. 3; and
- FIG. 6: is a diagram illustrating scan lines of a probe and inspection areas of the scan lines when the ultrasonic diagnostic device of FIG. 1 is used with the movement limiting instrument.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

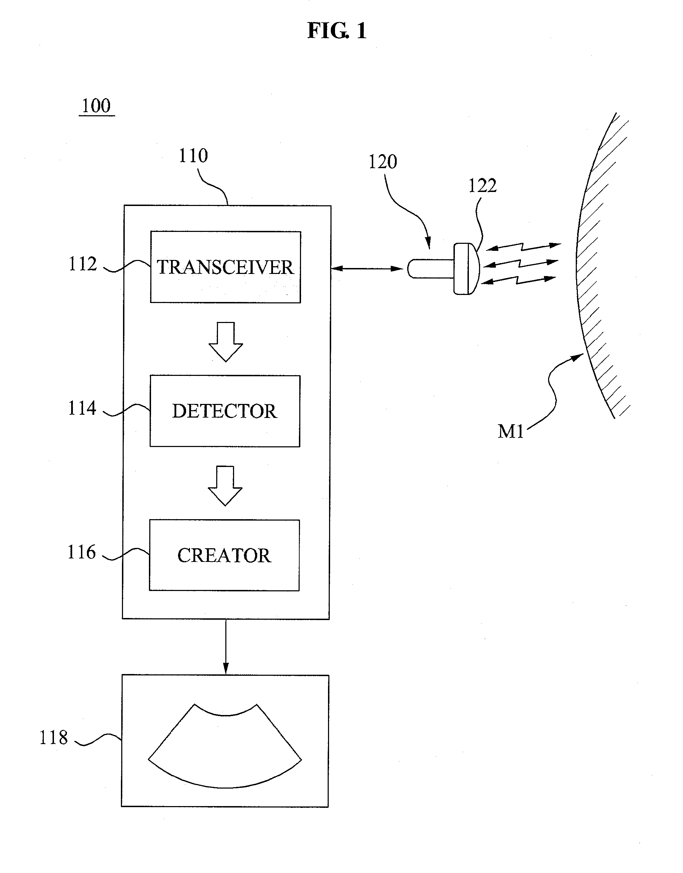

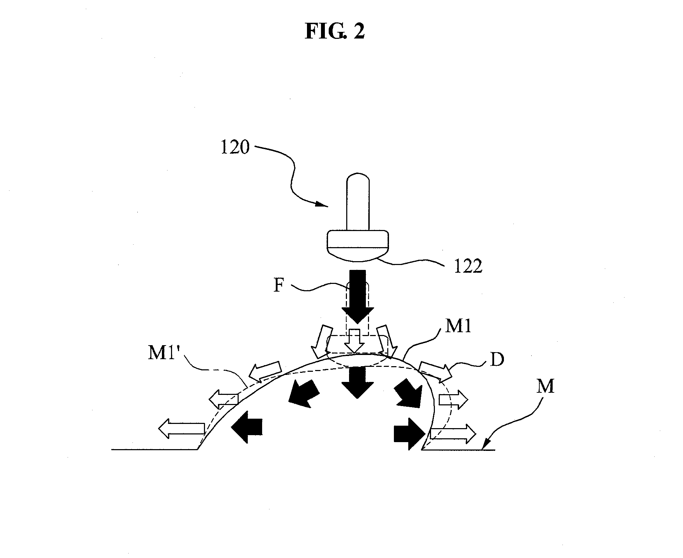

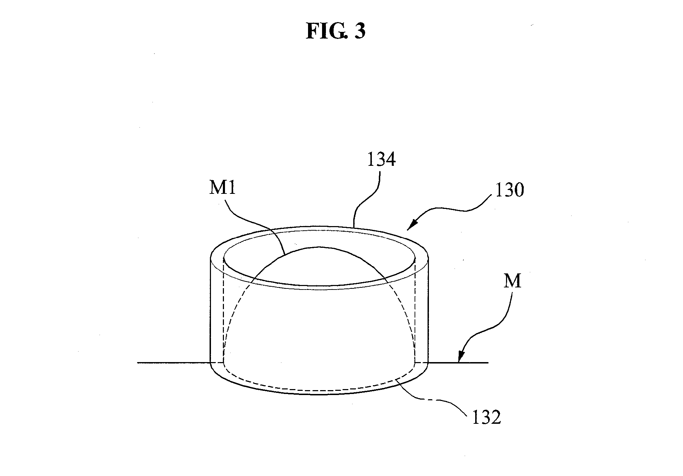

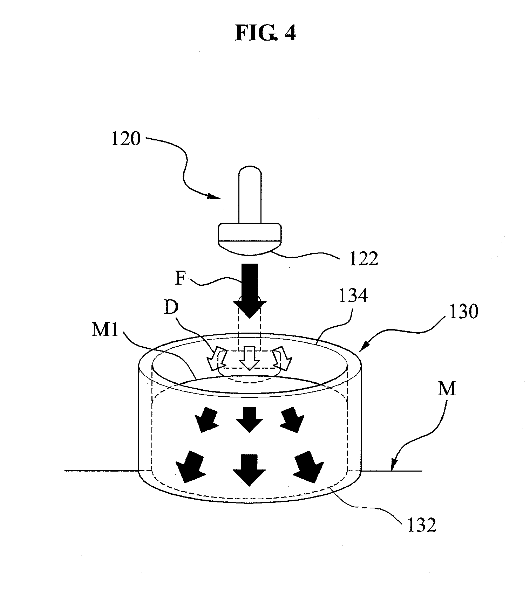

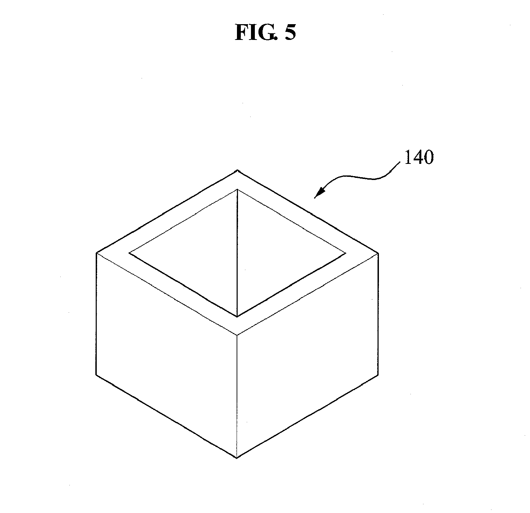

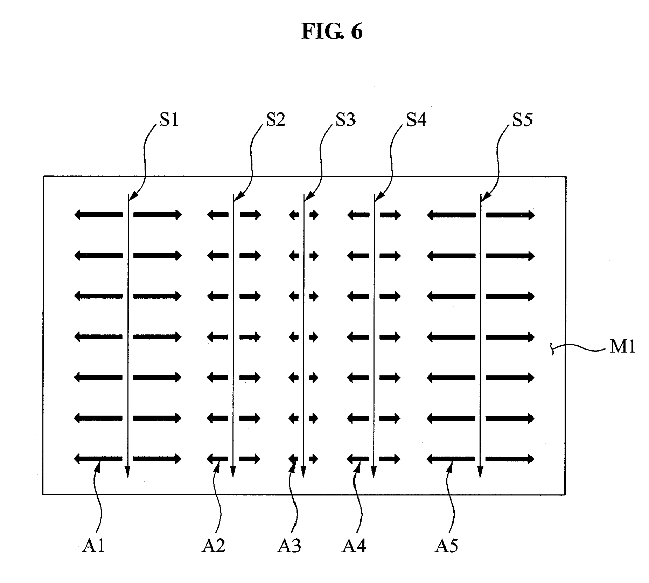

FIG. 1 is a diagram illustrating a configuration of an ultrasonic diagnostic device 100 according to an embodiment of the present invention, FIG. 2 is a view for describing an operational state when the ultrasonic diagnostic device 100 of FIG. 1 is used without a movement limiting instrument 130, and FIG. 4 is a view for describing an operational state when the ultrasonic diagnostic device 100 is used with the movement limiting instrument 130. FIG. 3 is a view for describing a disposed state of the movement limiting instrument 130 when the ultrasonic diagnostic device 100 is used with the movement limiting instrument 130, FIG. 5 is a perspective view for describing another example of the movement limiting instrument 130 of FIG. 3, and FIG. 6 is a diagram illustrating scan lines of a probe 120 and inspection areas of the scan lines when the ultrasonic diagnostic device 100 is used with the movement limiting 130.

Referring to FIGS. 1, 3, and 4, the ultrasonic diagnostic device 100 according to an embodiment of the present invention may include a body 110, the probe 120, and the movement limiting instrument 130. The body 110 may inspect an inspection portion of a subject M using ultrasonic signals. The probe 120 may transmit and receive the ultrasonic signals to and from the subject M. When an ultrasonic diagnosis is performed, the probe 120 may be connected with the body 110 to contact with an inspection portion M1 of the subject M. The movement limiting instrument 130 corresponds to a member limiting an abnormal elastic movement of the inspection portion M1 of the subject M. When the ultrasonic diagnosis is performed, the movement limiting instrument 130 may be attached onto the inspection portion M1 of the subj ect M.

Referring to FIG. 1, the body 110 may include a transceiver 112, a detector 114, and a creator 116.

The transceiver 112 may control ultrasonic signals transmitted from the probe 120, and may also receive response signals received by the probe 120. The transceiver 112 may be connected with the probe 120 in a wired or wireless manner. The transceiver 112 may control a magnitude, a direction, a time, and the like of the ultrasonic signals.[not in claims] Also, the transceiver 112 may transfer the response signals, that is, the ultrasonic signals reflected from an internal tissue of the subject M, to the detector 114.

The detector 114 may detect image information associated with the inspection portion M1 of the subject from the transferred response signals. Specifically, the detector 114 may calculate data of the response signals transferred from the transceiver 112, and detect image information associated with the internal tissue of the inspection portion M1 of the subject M.

The creator 116 may create an actual image of the internal tissue of the subject M from the image information of the detector 114. Specifically, the creator 116 may configure the image associated with the internal tissue of the inspection portion M1 of the subject M based on the detected image information.

The creator 116 may display the image of the inspection portion M1 on a separately provided display unit 118. The display unit 118 may be included in the body 110, and may also be provided separately from the body 110. Hereinafter, the present invention will be described based on a case where the display unit 118 is included in the body 110. The display unit 118 may be provided as a display device, for example, a liquid crystal display (LCD), a light-emitting diode (LED) monitor, and a cathode ray tube.

Referring to FIGS. 1 and 4, the probe 120 may include a plurality of transducers (not shown) on a contact portion 122 contacting with the subject M. The probe 120 may transmit and receive the ultrasonic signals using the transducers. The probe 120 may be provided in a shape that a user may easily grasp. When the ultrasonic diagnosis is performed, the probe 120 may move along the inspection portion M1 of the subject M with tightly attaching the contact portion 122 on the inspection portion M1 of the subject M.

Referring to FIGS. 1 and 6, the transducers may be arranged on the contact portion 122. The transducers arranged on the contact portion 122 may form a plurality of scan lines S1, S2, S3, S4, and S5. Here, the scan lines S1, S2, S3, S4, and S5 correspond to reference lines for the probe 120 to transmit and receive the ultrasonic signals.

The scan lines S1, S2, S3, S4, and S5 may be extended along a movement direction of the probe 120. The scan lines S1, S2, S3, S4, and S5 may be separated away from each other in a direction orthogonal to the movement direction of the probe 120. Hereinafter, the present invention will be described based on a case where the scan lines S1, S2, S3, S4, and S5 are provided in parallel at regular intervals, however, the scan lines S1, S2, S3, S4, and S5 may be provided in parallel at different intervals depending on a design condition. Also, for ease of description, the movement direction of the probe 120 is limited to a front direction and a rear direction, and the direction orthogonal to the movement direction of the probe 120 is limited to the left and the right.

Inspection areas A1, A2, A3, A4, and A5 may be formed on the scan lines S1, S2, S3, S4, and S5. The inspection areas A1, A2, A3, A4, and A5 may be formed in the same range on the left and the right of the scan lines S1, S2, S3, S4, and S5, respectively. However, depending on the design condition of the ultrasonic diagnostic device 100, the inspection areas A1, A2, A3, A4, and A5 may be provided to have different ranges on the left and the right of the scan lines S1, S2, S3, S4, and S5, respectively, or may also be provided on either the left or the right of the scan lines S1, S2, S3, S4, and S5, respectively.

The detector 114 may detect image information associated with the inspection portion M1 of the subject M using all the data of the response signals received by the scan lines S1, S2, S3, S4, and S5. Here, when a great amount of data is included in the response signals, a data processing time may significantly increase and thus a high performance calculation device may be required. Accordingly, the detector 114 may calculate image information associated with the internal tissue of the subject M, based on only data with respect to the inspection areas A1, A2, A3, A4, and A5 that are formed on the scan lines S1, S2, S3, S4, and S5. The detector 114 may obtain image information associated with other areas excluding the inspection areas A1, A2, A3, A4, and A5 by estimating and approximating the image information associated with the inspection areas A1, A2, A3, A4, and A5 according to various schemes.

More specifically, when the scan lines S1, S2, S3, S4, and S5 of the probe 120 inspect the inspection portion M1 of the subject M and then transfer all the inspected data to the body 110, the detector 114 of the body 110 may process a great amount of image information data. Accordingly, it may take long time to detect image information of the subject M via the detector 114. Due to the data processing delay of the detector 140, the creator 116 may not provide an image in real time. Accordingly, loads of the detector 114 may be prevented by reducing the inspection areas A1, A2, A3, A4, and A5 of the scan lines S1, S2, S3, S4, and S5 while maintaining sufficient image information associated with the internal tissue of the subject M.

Referring to FIGS. 3 through 5, the movement limiting instrument 130 may limit the abnormal elastic movement of the inspection portion M1 of the subject M occurring due to a physical pressure F of the probe 120 acting on the inspection portion M1 of the subject M. For example, the movement limiting instrument 130 may be effectively used when a breast cancer diagnosis is performed for a breast portion that has a high fluidity and has a protruded form.

The movement limiting instrument 130 may be formed of a non-transformable solid material to stably support the inspection portion M1 of the subject M. For example, the movement limiting instrument 130 may be formed of a metal, a plastic, a glass, or a wood. The movement limiting instrument 130 may be provided in a cylindrical shape that has a vertically hollow inside. A subject opening 132 may be formed in a lower portion of the movement limiting instrument 130. The inspection portion M1 of the subject M may be inserted into the subject opening 132. A probe opening 134 may be formed in an upper portion of the movement limiting instrument 130 so that the probe 120 may be movable.

Specifically, when the inspection portion M1 of the subject M is inserted into the subject opening 132, the inspection portion M1 of the subject may be immobilized and fixed within the movement limiting instrument 130. When the probe 120 is disposed in the probe opening 134, the probe 120 may movably contact with the inspection portion M1 of the subject M that is disposed within the movement limiting instrument 130.

Also, a section of the movement limiting instrument 130 may be provided in various shapes. For example, the movement limiting instrument 130 may have a section in a circular shape, an oval shape, a polygonal shape, or a closed-curved shape. Also, the section of the movement limiting instrument 130 may be provided in various shapes according to a shape of the inspection portion M1 of the subject M.

For example, FIG. 5 illustrates a movement limiting instrument 140 having a section in a rectangular shape. When the section of the movement limiting instrument 140 is formed in the rectangular shape, a shape of the subject opening 132 and the probe opening 134 may be provided in the rectangular shape. Coordinates of the subject M and the probe 120 may be very easily set. Accordingly, an operation algorithm of the ultrasonic diagnostic device 100 may be further conveniently designed. Hereinafter, the present invention will be described based on the movement limiting instrument 130 in the circular shape as shown in FIGS. 3 and 4.

Referring to FIGS. 1, 4, and 6, when the ultrasonic diagnosis is performed, the movement limiting instrument 130 may stably support the inspection portion M1 of the subject M and thereby prevent the abnormal elastic movement of the inspection portion M1 of the subject M occurring due to the contact pressure F of the probe 120. However, the movement limiting instrument 130 may not completely prevent the elastic movement of the inspection portion M1 of the subject M. For example, in the case of breast cancer diagnosis, the elastic movement may slightly occur in the inspection portion M1 of the subject M supported by the movement limiting instrument 130, due to the contact pressure F of the probe 120. The elastic movement of the inspection portion M1 of the subject M may be insignificant in comparison to a case where the movement limiting instrument 130 is unused. However, there is a need to perform a compensation in order to enhance a reliability and accuracy of the ultrasonic diagnostic device 100.

For this, the body 110 may extend the inspection areas A1, A2, A3, A4, and A5 of the scan lines S1, S2, S3, S4, and S5 in proportion to distances between the center of the probe 120 and the scan lines S1, S2, S3, S4, and S5. A relatively greater abnormal elastic movement of the inspection portion M1 of the subject M may occur as the subject is further from the center of the probe 120. Specifically, when the probe 120 is tightly attached onto the inspection portion M1 of the subject M, an elastic movement may occur in the inspection portion M1 of the subject M from the center of the probe 120 towards a side direction. The abnormal elastic movement of the subject M barely occurs in the center of the probe 120, however, may become greater as the subject M is further from the center of the probe 120.

As the distances between the center of the probe 120 and the scan lines S1, S2, S3, S4, and S5 increase, the inspection areas A1, A2, A3, A4, and A5 of the scan lines S1, S2, S3, S4, and S5 may be extended. Specifically, since the relatively greater abnormal elastic movement of the inspection portion M1 of the subject M occurs as the subject M is further from the center of the probe 120, the inspection areas A1, A2, A3, A4, and A5 of the scan lines S1, S2, S3, S4, and S5 may be further extended as the scan lines S1, S2, S3, S4, and S5 are further from the center of the probe 120. Therefore, a displacement D of the internal tissue according to the elastic movement amount of the subject M may be sufficiently detected. It is possible to compensate for a distortion in image information associated with the internal tissue from the detected displacement.

The scan lines S1, S2, S3, S4, and S5 may be symmetrically disposed on the left and the right of the contact portion 122 based on the center of the contact portion 122. Accordingly, sizes of the inspection areas A1, A2, A3, A4, and A5 of the scan lines S1, S2, S3, S4, and S5 may be symmetrically formed on the left and the right of the contact portion 122 based on the center of the contact portion 122.

Hereinafter, an operation of the ultrasonic diagnostic device 100 constructed as above will be described.

Referring to FIGS. 3 and 4, the movement limiting instrument 130 may be disposed on the inspection portion M1 of the subject M that the user desires to inspect. Here, the inspection portion M1 of the subjected M is inserted into the subject opening 132 of the movement limiting instrument 130.

When attaching of the movement limiting instrument 130 is completed, the body 110 may be operated. The probe 120 may be disposed on the inspection portion M1 of the subject M through the probe opening 134.

The probe 120 may closely contact with the inspection portion M1 of the subject M at a predetermined pressure F, and move along the surface of the inspection portion M1 of the subject M. Here, the probe 120 may transmit and receive ultrasonic signals to the inside of the inspection portion M1 of the subject M.

Since the inspection portion M1 of the subject M is supported in a state where its movement is limited by the movement limiting instrument 130, the contact pressure F of the probe 120 may barely move the inspection portion M1 of the subject M towards the side direction. Accordingly, the displacement D may barely occur in the inspection portion M1 of the subject M in the direction orthogonal to the contact pressure F of the probe 120. The abnormal elastic movement of the inspection portion M1 of the subject M may barely occur.

In the case of the above slight elastic movement of the inspection portion M1 of the subject M occurring when the movement limiting instrument 130 is used, it is possible to compensate for a distortion in image information by adjusting the inspection areas A1, A2, A3, A4, and A5 of the scan lines S1, S2, S3, S4, and S5 formed on the probe 120 as a displacement D of the internal tissue according to the elastic movement amount of the subject M, and using the response signals of the inspection areas A1, A2, A3, A4, and A5. Specifically, even when the subject M includes an elastically moving property, it is possible to stably obtain an image of internal tissue when the ultrasonic diagnosis is performed.

The received response signals may be transferred to the detector 114 via the transceiver 112. The detector 114 may detect image information associated with the inspection portion M1 of the subject M from the transferred response signals. The creator 116 may create the image of the internal tissue of the subject M from the image information. The image of the subject M may be displayed on the display unit 118.

FIG. 2 illustrates a case where the movement limiting instrument 130 is unused when the ultrasonic diagnosis is performed using the ultrasonic diagnostic device 100. When the contact pressure F of the probe 120 acts on the inspection portion M1 of the subject M, all the contact pressure F of the probe 120 may be distributed into every direction. Therefore, an inspection portion M1' of the subject M may be being elastically pushed and whereby the displacement D spreading towards the sides may occur. Due to the elastic movement of the inspection portion M1' of the subject M, image information associated with an internal tissue of the inspection portion M1' detected by the probe 120 may be distorted.

According to an embodiment of the present invention, when an ultrasonic diagnosis is performed, an ultrasonic diagnostic device may include a movement limiting instrument on an inspection portion of a subject to thereby prevent an abnormal elastic movement of the inspection portion of the subject due to a contact pressure of a probe.

Also, according to an embodiment of the present invention, it is possible to embody a simple change in a configuration by including a movement limiting instrument to prevent an abnormal elastic movement of an inspection portion of a subject.

Also, according to an embodiment of the present invention, it is possible to prevent an abnormal elastic movement of an inspection portion of a subject occurring due to a contact pressure of a probe. Therefore, it is possible to enhance a diagnostic performance of an ultrasonic diagnostic device, and to decrease an erroneous diagnosis probability of the ultrasonic diagnostic device.

Also, according to an embodiment of the present invention, when an abnormal elastic movement of an inspection portion of a subject occurs, inspection areas of scan lines may be extended in proportion to distances between a center of a probe and the scan lines. Therefore, it is possible to effectively compensate for a change in an elastic movement amount of an inspection portion of the subject according to a contact location of the probe. Also, it is possible to prevent an increase in an amount of data used for an image creation by appropriately adjusting the inspection areas of the scan lines.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. An ultrasonic diagnostic device comprising:
a body inspecting a subject using an ultrasonic signal;
a probe being connected with the body to contact with an inspection portion of the subject, and to transmit and receive the ultrasonic signal to and from the subject; and
a movement limiting instrument being disposed on the inspection portion of the subject to limit an abnormal elastic movement of the inspection portion of the subject occurring due to a contact pressure of the probe, when an ultrasonic inspection is performed for the subject.

2. The ultrasonic diagnostic device of claim 1, wherein the movement limiting instrument is provided in a cylindrical shape that has a vertically hollow inside.

3. The ultrasonic diagnostic device of claim 2, wherein the movement limiting instrument has a section in a circular shape, an oval shape, a polygonal shape, or a closed-curved shape.

4. The ultrasonic diagnostic device of claim 2, wherein:
a subject opening is formed in a lower portion of the movement limiting instrument, and the inspection portion of the subject is inserted into the subject opening, and
a probe opening is formed in an upper portion of the movement limiting instrument to inspect the inspection portion of the subject inserted into the
subject opening using the probe.

5. The ultrasonic diagnostic device of claim 4, wherein:
the movement limiting instrument has a section in a circular shape, an oval shape, a polygonal shape, or a closed-curved shape, and
the subject opening and the probe opening are provided in the same shape as the section of the movement limiting instrument to be connected through each other.

6. The ultrasonic diagnostic device of claim 1, wherein the movement limiting instrument is formed of a solid material to stably limit an elastic movement of the inspection portion of the subject.

7. The ultrasonic diagnostic device of claim 1, wherein the body comprises:
a transceiver controlling the ultrasonic signal transmitted from the probe, and receiving a response signal received by the probe;
a detector detecting image information associated with the inspection portion of the subject from the response signal transferred from the transceiver; and
a creator to create an image of an internal tissue of the subject from the detected image information.

8. The ultrasonic diagnostic device of claim 7, wherein:
the probe forms, on the inspection portion of the subject, a plurality of scan lines that is extended along a movement path, and
the detector sets the plurality of scan lines to have different sizes of inspection areas in order to compensate for the abnormal elastic movement of the inspection portion of the subject.

9. The ultrasonic diagnostic device of claim 8, wherein the plurality of scan lines is provided in parallel at regular intervals.

10. The ultrasonic diagnostic device of claim 8, wherein the inspection areas of the scan lines are formed on both sides of the scan lines, respectively.

11. The ultrasonic diagnostic device of claim 8, wherein the inspection areas of the scan lines are extended in proportion to distances between a center of the probe and the scan lines.
